# FASCICULE DE BREVET EUROPEEN

(11) **EP 2 453 849 B1**
(45) Date de publication et mention de la délivrance du brevet: **12.11.2014**
(21) Numéro de dépôt: 10752021.5
(22) Date de dépôt: 15.07.2010
(51) Int. Cl.: A61F 5/01

(54) **Orthèse pour le traitement de la rhizarthrose**
Orthese zur Behandlung von Rhizarthrose
Orthosis for treating rhizarthrosis

(30) Priorité: 17.07.2009 FR 0903534
(43) Date de publication de la demande: 23.05.2012
(73) Titulaire: Gibaud, 42000 Saint Etienne (FR)
(72) Inventeur: ANGLADA, Gérard, F-42000 Saint Etienne (FR); BECKERS, Thomas, IS-210 Gardabaer (IS)
(74) Mandataire: Delorme, Nicolas
(86) Numéro de dépôt international: PCT/FR2010/051489
(87) Numéro de publication internationale: WO 2011/007103

(56) Documents cités:
- WO-A1-2006/117808
- FR-A1- 2 822 371
- FR-A1- 2 854 564
- US-B1- 6 496 984

## Description

La présente invention concerne une orthèse destinée à être placée sur la main d'un utilisateur, notamment pour le traitement de la rhizarthrose.

La rhizarthrose - ou arthrose de la base du pouce - est l'usure du cartilage de l'articulation à la base du pouce, entre le trapèze et l'os métacarpien. Outre les douleurs qu'elle occasionne, cette pathologie peut entraîner un enraidissement et une déformation du pouce empêchant de saisir un objet entre le pouce et l'index ou les autres doigts. Il existe également un risque de luxation de l'articulation.

Le traitement de la rhizarthrose par la voie orthopédique vise d'une part à diminuer la douleur et d'autre part à éviter la perte de l'amplitude articulaire, c'est-à-dire l'impossibilité d'écarter le pouce de l'index.

On connaît déjà plusieurs types d'orthèses de la main utilisées pour le traitement de la rhizarthrose.

Selon une première réalisation connue, une telle orthèse consiste en une pièce rigide réalisée en un matériau thermoformable. L'intervention d'un orthésiste est généralement requise, pour réaliser cette pièce sur mesure. Une orthèse de ce type s'avère donc contraignante et coûteuse à réaliser. De plus, elle ne peut être facilement adaptée aux conditions de port, typiquement au port diurne et au port nocturne.

Selon une deuxième réalisation connue, une telle orthèse consiste en une sangle textile, éventuellement pourvue de raidisseurs, que l'utilisateur vient enrouler et fixer autour de sa main. Cette orthèse est généralement difficile à mettre en place par l'utilisateur seul. De plus, un mauvais positionnement peut nuire au traitement orthopédique.

Une autre orthèse de main pour le traitement de la rhizarthrose est proposée dans le document WO 2006/117808. L'orthèse décrite dans ce document présente un certain nombre d'inconvénients.

Tout d'abord, elle est d'une mise en place peu aisée, en particulier lorsqu'il s'agit de positionner la bande additionnelle entourant le pouce, qui est nécessaire au maintien de la pièce sur laquelle repose le pouce.

D'autre part, afin d'obtenir une immobilisation efficace de la zone de la lésion, cette orthèse bloque d'autres zones alors que cela ne s'avère pas directement nécessaire pour la pathologie concernée. Il s'agit en particulier la partie distale du pouce, de la base des doigts longs et d'une zone étendue autour de l'articulation du poignet. Ceci occasionne une limitation importante des mouvements de la main, ce qui rend l'orthèse peu confortable, et peu pratique et peut dissuader certaines personnes de la porter.

Le document FR 2 854 564 décrit une orthèse comportant un moyen élastique en U qui est placé entre le pouce et l'index et épouse la forme de la première commissure.

La présente invention vise à remédier aux inconvénients mentionnés ci-dessus, en fournissant une orthèse confortable, simple d'utilisation, efficace, et avantageusement modulable.

A cet effet, l'invention propose une orthèse conforme à la revendication 1.

Ainsi, l'organe de maintien assure un écartement entre le pouce et l'index qui procure un effet antalgique et préserve l'amplitude articulaire. En outre, la sangle crée, en combinaison avec l'organe de maintien, un appui de part et d'autre de la main qui immobilise la colonne ostéo-articulaire du pouce et empêche la luxation de l'articulation.

Grâce à l'orthèse selon l'invention, l'os métacarpien du pouce est immobilisé dans toutes les directions, à savoir :
- d'une part dans le sens du rapprochement du pouce vers l'index, au moyen de l'organe de maintien en selle de cheval. Cet organe forme une gorge en U ouverte vers le poignet qui prend appui sur le fond de la commissure, et enveloppe la phalange proximale du pouce et au moins la partie proximale de la phalange proximale de l'index, limitant ainsi le déplacement du pouce. De plus, ces phalanges sont localement emprisonnées entre la bande de jonction et les ailes de l'organe de maintien ;
- d'autre part dans le sens de la prono-supination, au moyen de la sangle et de l'organe de maintien, grâce auxquels l'os métacarpien est pris en sandwich.

Le fait que l'os métacarpien est emprisonné et immobilisé dans l'orthèse selon l'invention présente de nombreux avantages.

En particulier, l'orthèse permet d'immobiliser l'articulation trapézo-métacarpienne, qui est le siège de la lésion, et ainsi d'obtenir directement l'effet recherché. L'immobilisation directe de la zone de la lésion implique qu'il n'est pas nécessaire d'immobiliser d'autres zones de la main.

Ainsi, d'une part, il n'est pas nécessaire de prévoir en plus une bande circulaire entourant le pouce. De ce fait, l'orthèse selon l'invention est plus simple à mettre en place, les risques de garrot sont évités, la colonne du pouce est libérée, et la pulpe du pouce est également libérée - ce qui est très appréciable pour l'utilisateur qui conserve les sensations de toucher au quotidien.

D'autre part, la sangle de l'orthèse selon l'invention peut être moins enveloppante, et notamment libérer la base des doigts longs donc ne pas gêner leur mouvement, notamment en extension.

En pratique, selon un premier mode de réalisation, l'organe de maintien est simplement déposé dans la commissure pouce-index et le cas échéant appliqué contre celle-ci, puis la sangle est enveloppée autour de la main et de l'organe de maintien. La mise en place de l'orthèse est particulièrement simple, et sans risque de mauvais positionnement du fait notamment de la forme ergonomique de l'organe de maintien. La combinaison de l'organe de maintien et de la sangle assure une bonne efficacité de l'orthèse sans pour autant nuire au confort de port.

Selon un deuxième mode de réalisation, l'organe de maintien est déjà associé à la sangle, celle-ci formant un manchon qui s'enfile comme un gant. Après enfilage sur la main, une partie libre de la sangle est rabattue contre la face dorsale de la main avec le serrage approprié.

Avantageusement, l'organe de maintien et la sangle sont deux pièces distinctes. Ainsi, l'orthèse est modulable. En effet, l'organe de maintien étant amovible, la sangle peut s'utiliser seule, par exemple en port diurne.

Selon une réalisation possible, l'aile interne de l'organe de maintien est courbée vers l'extérieur en direction de son extrémité libre. La forme évasée de l'aile externe favorise la mise en place de l'organe de maintien entre le pouce et l'index. De plus, cela permet de renforcer l'appui contre la paume de la main, par l'élasticité ainsi procurée à l'organe de maintien.

L'organe de maintien peut être réalisé en une matière souple, telle que le silicone ou le SEBS (styrène éthylbutylène styrène). Il est alors particulièrement confortable, tout en présentant la tenue nécessaire à sa fonction, en particulier d'écartement du pouce. De plus, il peut épouser la forme de la commissure et assurer un bon appui dans le creux entre le pouce et l'index.

En variante ou en complément, l'organe de maintien peut comporter une pièce en U rigide située sensiblement à la jonction entre les ailes interne et externe, ladite pièce en U pouvant être déformée de façon à modifier l'écartement entre le pouce et l'index selon la morphologie de l'utilisateur. L'organe de maintien peut par exemple être moulé autour de cette pièce en U.

En outre, l'orthèse peut comprendre une tige rigide associée à l'organe de maintien et/ou à la sangle de sorte que, en position d'utilisation, ladite tige s'étende sur la face dorsale de la main sensiblement dans le prolongement de l'aile externe de l'organe de maintien et crée un appui contre la face dorsale de la main. L'appui créé par cette tige permet un très bon maintien du métacarpien, empêchant ainsi la luxation de l'articulation. Cette tige dorsale, qui est de préférence emboîtée dans l'organe de maintien, complète l'emprisonnement du métacarpien et de la phalange proximale du pouce dans l'orthèse. Cette tige, maintenue sur la main par la sangle, interdit les mouvements de flexion, extension, inclinaison radiale et inclinaison cubitale.

Avantageusement, la tige rigide peut être réalisée en un matériau déformable de sorte qu'une personne puisse la plier ou l'incurver de façon à placer le poignet en légère flexion, en position d'utilisation de l'orthèse. Selon les besoins, le poignet peut être placé en flexion palmaire ou en dorsiflexion. La tige est par exemple réalisée en aluminium.

On peut prévoir que l'organe de maintien comprenne un logement ménagé dans son aile externe et destiné à recevoir une partie extrême de la tige rigide. L'autre partie extrême de la tige peut être située dans un logement ménagé dans la sangle ou, en variante, être simplement emprisonnée entre deux pans de la sangle.

Selon une réalisation possible, la sangle présente, à plat, sensiblement la forme d'un T qui possède une première et une deuxième branches sensiblement dans le prolongement l'une de l'autre destinées à envelopper la main en étant rabattues contre la face dorsale de la main, et une troisième branche destinée à être rabattue vers la face dorsale de la main en passant par la commissure pouce-index et en recouvrant l'organe de maintien. La troisième branche permet de réaliser un réglage en pression de l'organe de maintien. De plus, cette forme en T permet de dégager la face dorsale de la main en libérant les métacarpo-phalangiennes du majeur, de l'annulaire et de l'auriculaire.

En outre, la sangle peut présenter une dimension suffisante pour entourer également le poignet, en position d'utilisation. L'immobilisation du poignet permet de créer un bras de levier important qui améliore l'effet de l'orthèse sur la rhizarthrose. Ceci peut être particulièrement utilisé en port nocturne, tandis qu'une sangle plus courte, laissant le poignet libre, peut être utilisée en port diurne.

La sangle peut comporter au moins un élément de rigidification qui, en position d'utilisation, s'étend sur la face dorsale de la main sensiblement dans le prolongement de l'aile externe de l'organe de maintien. Il peut s'agir par exemple d'une baleine engagée dans un fourreau de la sangle. Ceci permet de créer un complément d'appui contre la face dorsale de la main.

Avantageusement, l'orthèse peut être agencée pour que, lorsqu'elle est portée par un utilisateur, la main de l'utilisateur équipée de l'orthèse ait une légère inclinaison cubitale, ce qui permet de diminuer la tension des muscles du poignet. Cet effet peut être obtenu au moyen de la tige rigide précitée.

L'orthèse peut en outre comprendre une barrette rigide associée à la sangle, ladite barrette s'étendant, en position d'utilisation, du côté intérieur de la main et de l'avant-bras sensiblement selon l'axe de l'avant-bras, présentant une longueur suffisante pour s'étendre de part et d'autre de l'articulation du poignet, et possédant une forme courbée épousant le relief du poignet et de la paume de la main. Cette barrette palmaire permet de compléter l'action d'immobilisation du poignet et de la colonne du pouce.

La combinaison de l'organe de maintien, de la tige dorsale et de la barrette palmaire, associés à la sangle, offre une immobilisation par trois points d'appui particulièrement efficace.

On décrit à présent, à titre d'exemples non limitatifs, plusieurs modes de réalisation possibles de l'invention, en référence aux figures annexées :
Les figures 1 à 3 représentent un organe de maintien appartenant à l'orthèse selon l'invention, respectivement en vue externe, en vue interne, et en vue latérale ;
Les figures 4 et 5 représentent l'organe de maintien placé sur la main gauche d'un utilisateur, respectivement en vue externe et en vue interne ;
La figure 6 est une vue schématique, à plat, d'une sangle appartenant à l'orthèse selon l'invention ;
La figure 7 est une vue en perspective d'une tige rigide pouvant appartenir à l'orthèse selon l'invention ;
La figure 8 est une vue externe de la main droite d'un utilisateur équipée de l'organe de maintien, avec la sangle en cours de mise en place ;
Les figures 9 et 10 sont des vues de la main d'un utilisateur équipée de l'orthèse, respectivement la main droite en vue externe et la main gauche en vue interne ;
La figure 11 est une vue externe de la main gauche d'un utilisateur équipée de l'orthèse, montrant l'inclinaison cubitale de la main ;
La figure 12 est une vue en perspective d'une pièce rigide en U pouvant appartenir à l'orthèse selon l'invention ;
La figure 13 représente un organe de maintien comportant la pièce rigide en U de la figure 12 ;
La figure 14 est une vue interne de la main droite d'un utilisateur équipée de l'orthèse pourvue en outre d'une barrette rigide ;
Les figures 15 à 18 sont différentes vues de la main droite d'un utilisateur équipée d'une orthèse selon l'invention ;
Les figures 19 à 22 sont des vues similaires aux figures 15 à 18, respectivement, la sangle e l'orthèse n'étant pas représentée.

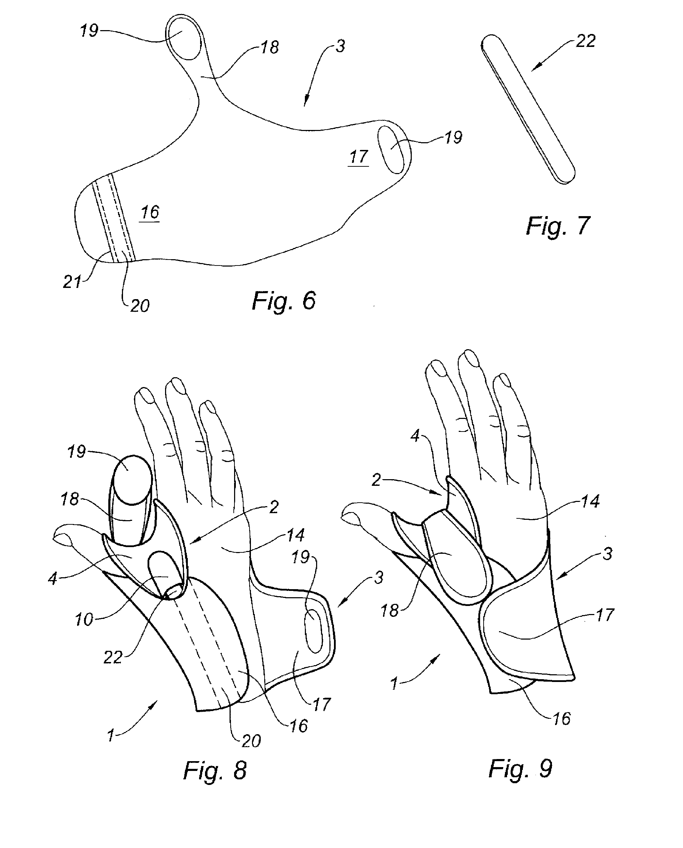

Une orthèse 1 selon l'invention, comme illustrée sur les figures 8 à 11, comporte un organe de maintien 2 et une sangle 3.

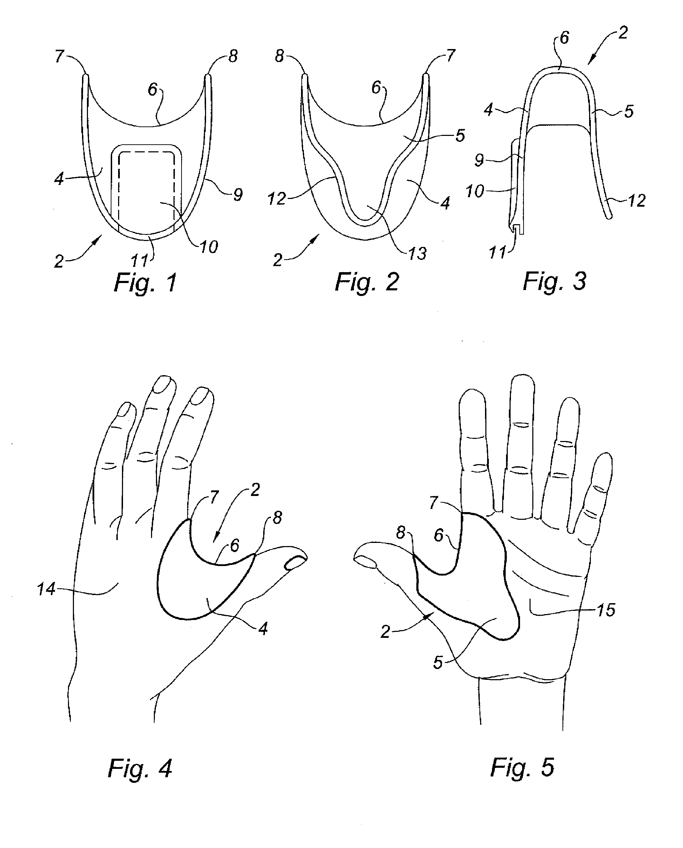

L'organe de maintien 2, illustré sur les figures 1 à 3, présente la forme générale d'une selle de cheval (forme de type paraboloïde hyperbolique). Ainsi, il possède une aile externe 4 et une aile interne 5 en vis-à-vis reliées par une bande de jonction 6 présentant une forme incurvée en U d'un bord extrême 7 à l'autre 8.

L'aile externe 4 est globalement plane et présente un bord libre 9 en forme de U joignant les deux bords extrêmes 7, 8 de la bande de jonction 6. Selon un mode de réalisation possible, l'aile externe 4 comporte de plus un logement 10 en saillie vers l'extérieur de l'organe de maintien 2 et possédant une ouverture 11 à l'opposé de la bande de jonction 6.

L'aile interne 5 présente un bord libre 12 qui joint les deux bords extrêmes 7, 8 de la bande de jonction 6 et comporte une languette centrale 13. L'aile interne 5 est globalement parallèle à l'aile externe 4 tout en étant courbée vers l'extérieur depuis la bande de jonction 6 en direction de son extrémité libre, comme on le voit sur la figure 3.

L'organe de maintien 2 est placé sur la main d'un utilisateur comme illustré sur les figures 4 et 5. L'organe de maintien est engagé entre le pouce et l'index, de part et d'autre de la main, la forme évasée de l'aile interne 5 facilitant cette mise en place, jusqu'à ce que la bande de jonction 6 repose sensiblement sur la commissure pouce-index. La main se trouve alors serrée entre l'aile externe 4, contre la face dorsale 14 de la main, et l'aile interne 5, contre la paume 15 de la main. De plus, la bande de jonction 6 prend appui d'une part sur la phalange proximale du pouce et d'autre part sur la phalange proximale de l'index, l'organe de maintien 6 formant une gouttière qui enveloppe partiellement ces phalanges. Ainsi, l'organe de maintien 2 permet d'une part de garantir un écartement entre le pouce et l'index et d'autre part d'assurer une pression de part et d'autre de la main, accentuée par la forme évasée de l'aile interne 5.

Dans la réalisation représentée, l'organe de maintien 2 est réalisé en une matière souple, telle que le silicone ou le SEBS (styrène éthylbutylène styrène). De ce fait, tout en présentant la tenue nécessaire à sa fonction de maintien de l'écartement, l'organe de maintien 2 peut être déformé élastiquement pour bien s'adapter à la commissure et en épouser la forme.

On décrit à présent la sangle 3 en référence à la figure 6.

La sangle 3 présente, à plat, sensiblement la forme d'un T qui possède une première et une deuxième branches 16, 17 sensiblement dans le prolongement l'une de l'autre et une troisième branche 18 orthogonale aux branches 16, 17. Avantageusement, la sangle 3 est réalisée à partir d'un tissu en maille 3D permettant le confort et la ventilation. La partie extrême des deuxième et troisième branches 17, 18 est pourvue d'une zone d'accrochage 19 de type Velcro ®. L'absence sur la sangle d'une branche de serrage comprimant la trapézo-métacarpienne est également un élément important pour l'amélioration du confort de port de l'orthèse 1.

En outre, la sangle 3 peut comporter une baleine 20 engagée dans un fourreau 21 prévu au niveau de la première branche 16, sensiblement parallèlement à la troisième branche 18. Ainsi, lorsque la sangle 3 est mise en place sur la main, la baleine 20 s'étend sur la face dorsale 14 de la main sensiblement dans le prolongement de l'aile externe 4 de l'organe de maintien 2.

L'orthèse 1 peut également comporter une tige rigide 22 ayant une forme plate et allongée comme illustré sur la figure 7. La tige rigide 22 est par exemple réalisée en aluminium.

En pratique, la mise en place de l'orthèse 1 sur la main s'effectue de la façon suivante. Tout d'abord, l'organe de maintien 2 est placé à la commissure pouce-index comme expliqué plus haut et comme illustré sur les figures 4 et 5. La tige rigide 22 est insérée dans le logement 10 ménagé sur l'aile externe 4 de l'organe de maintien, de sorte qu'elle s'étend alors sur la face dorsale 14 de la main sensiblement dans le prolongement de l'aile externe 4 de l'organe de maintien 2.

Puis la sangle 3 est placée autour de la main et de l'organe de maintien 2. Les première et une deuxième branches 16, 17 sont rabattues depuis la paume 15 contre la face dorsale 14 de la main, de sorte à envelopper la main, tandis que la troisième branche 18 est rabattue depuis la paume 15 vers la face dorsale 14 de la main en passant par la commissure pouce-index et en recouvrant l'organe de maintien 2 (voir figure 8). Les zones d'accrochage 19 permettent la fermeture de la sangle 3 ainsi que le réglage du serrage. Le fait d'avoir une sangle unique facilite grandement la mise en place de l'orthèse.

Dans cette position, la sangle 3 assure la retenue de l'organe de maintien 2 contre la commissure pouce-index et plaque la tige rigide 22 contre la face dorsale 14 de la main. L'os métacarpien du pouce est ainsi emprisonné dans l'orthèse 1 entre la sangle 3 et l'organe de maintien 2, que ce soit dans le plan des doigts, dans le sens de l'éloignement ou du rapprochement de l'index, ou dans la direction perpendiculaire à ce plan, vers la paume ou vers la face dorsale de la main. Un appui supplémentaire est créé par la tige rigide 22.

La tige rigide 22 peut être conservée rectiligne, afin de maintenir le poignet en position de repos (dans l'alignement du bras), ou être déformée, pour placer le poignet en position relevée (dorsiflexion) ou fléchie (flexion palmaire).

L'orthèse 1 est modulable et adaptable tant à la morphologie de l'utilisateur qu'au moment du port.

En effet, en port nocturne, il est préférable d'utiliser une sangle longue, immobilisant le poignet, et de porter l'organe de maintien 2. De ce fait, la nuit, l'orthèse 1 maintient le pouce dans une position d'écartement, préservant ainsi l'amplitude articulaire, ce qui permet de conserver une utilisation normale du pouce la journée. Par conséquent, en port diurne, la sangle 3 peut s'utiliser seule, c'est-à-dire sans l'organe de maintien 2. Il est également possible d'utiliser une sangle courte, laissant le poignet libre.

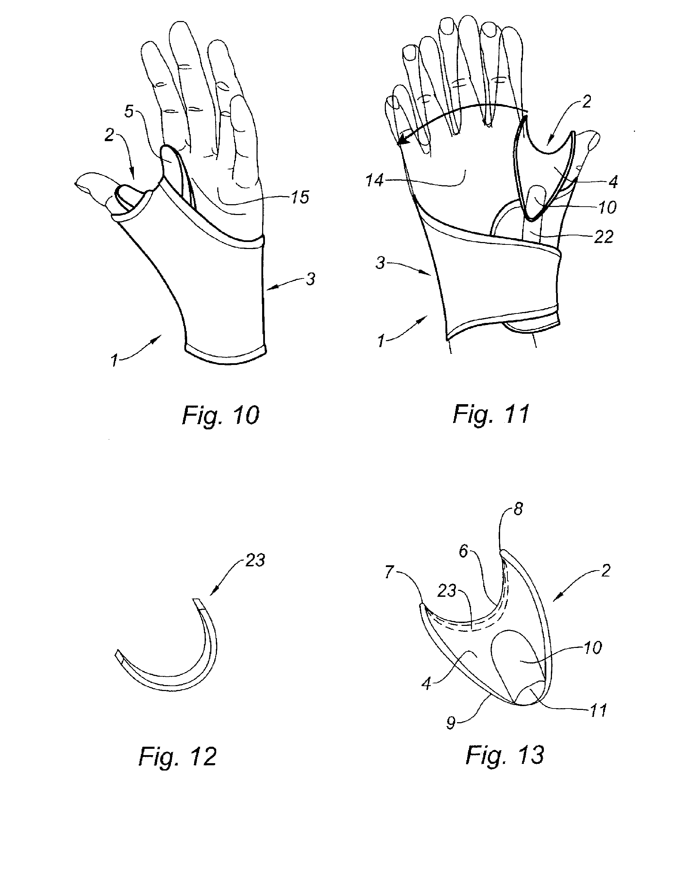

La figure 11 montre que la main équipée de l'orthèse 1 selon l'invention est avantageusement placée en légère inclinaison cubitale. Par exemple, c'est la rectitude de la tige rigide 22 qui maintient cette inclinaison cubitale, diminuant ainsi la tension des muscles du poignet.

Par ailleurs, comme on le voit sur les figures 9 et 11, l'orthèse 1 selon l'invention (à savoir l'organe de maintien 2, la sangle 3 et l'éventuelle tige rigide 22) peut être mise en place tant sur la main gauche que sur la main droite.

Selon une variante de l'invention, l'organe de maintien 2 comporte une pièce en U rigide 23 telle qu'illustrée sur la figure 12. Cette pièce 23 est par exemple réalisée en aluminium, et l'organe de maintien est moulé autour de cette pièce 23, comme on le voit sur la figure 13. La pièce 23 est située sensiblement à la jonction entre les ailes interne et externe 5, 4, en suivant la forme de la bande de jonction 6. La pièce 23 peut être déformée afin de modifier l'écartement entre le pouce et l'index selon la morphologie de l'utilisateur ou la gravité de la rhizarthrose. L'organe de maintien 2 peut alors parfaitement épouser la forme de la commissure.

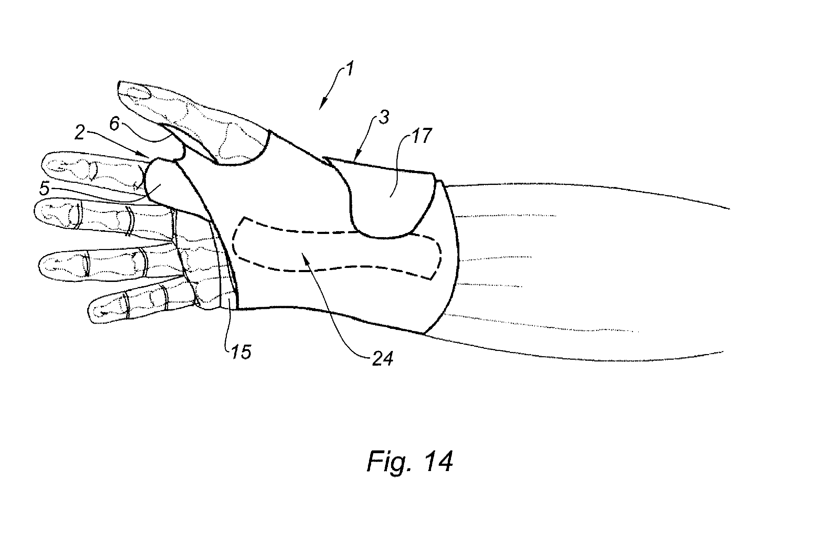

Comme illustré sur la figure 14, l'orthèse 1 selon l'invention peut en outre comprendre une barrette 24 rigide associée à la sangle 3. En position d'utilisation, cette barrette 24 s'étend du côté intérieur de la main et de l'avant-bras sensiblement selon l'axe de l'avant-bras. La barrette 24 présente une longueur suffisante pour s'étendre de part et d'autre de l'articulation du poignet, et possède une forme courbée épousant le relief du poignet et de la paume de la main.

La barrette 24 permet d'améliorer l'immobilisation du poignet. Elle est de préférence réalisée en aluminium, de façon à pouvoir être conformée manuellement pour placer le poignet en flexion palmaire ou en dorsiflexion.

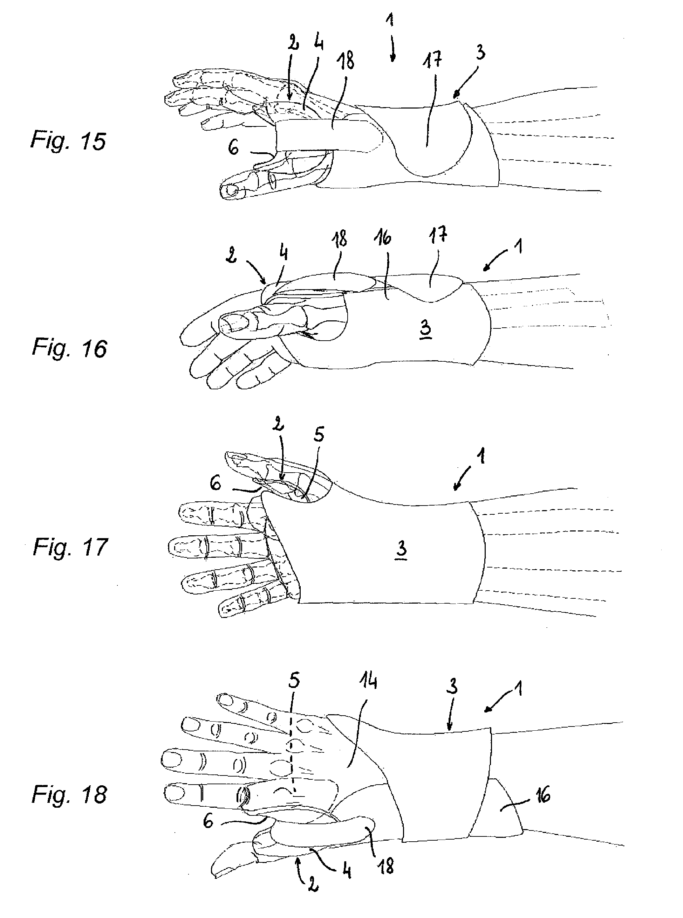

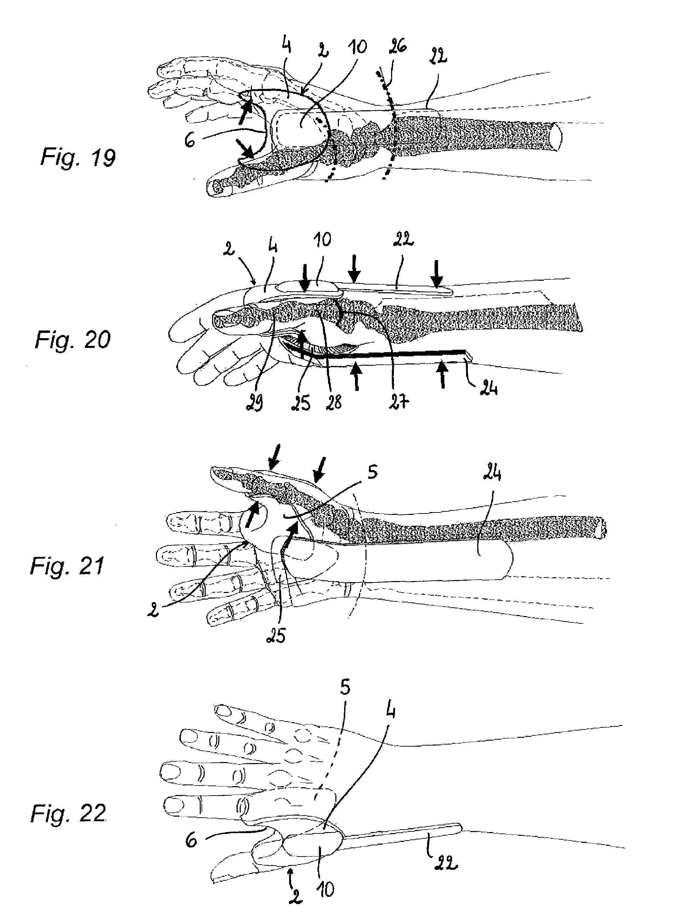

On se rapporte à présent aux figures 15 à 22, qui montrent bien l'effet d'immobilisation procuré par l'orthèse 1. Les flèches des figures 19 à 21 montrent les appuis qui contribuent à cette immobilisation. En particulier, la figure 19 illustre le maintien de l'ouverture de commissure et la figure 20 l'emprisonnement de la colonne du pouce entre la tige dorsale 22 et la barrette palmaire 24.

Sur ces figures, l'orthèse 1 comprend en outre un coussin palmaire 25. Il s'agit d'un élément optionnel qui permet de combler le creux entre la paume de la main et la sangle 3 - typiquement entre la paume de la main et la barrette palmaire 24 - et donc d'améliorer le confort.

Comme on le voit notamment sur les figures 19 et 20, l'orthèse 1 procure un emprisonnement du poignet (axe 26 d'articulation du poignet), de la trapézo-métacarpienne 27 qui est le siège de la lésion, du métacarpien 28 du pouce et de la phalange proximale 29 du pouce.

Dans ce mode de réalisation comportant le coussin 25, l'immobilisation de la trapézo-métacarpienne et du poignet est obtenue par la jonction du coussin palmaire 25 fixé sur la barrette palmaire 24 et de l'organe de maintien 6 fixé sur la tige dorsale 22.

Lorsque le pouce est en position de repos, il se situe dans le prolongement de l'avant-bras, ce qui permet une continuité entre le poignet et la colonne du pouce.

En outre, sur la figure 18, il est bien visible que la face dorsale 14 de la main est largement libérée, ce qui préserve le mouvement des doigts longs.

Ainsi, l'invention apporte une amélioration déterminante à la technique antérieure, en fournissant une orthèse particulièrement simple à mettre en place. Il suffit en effet de placer, le cas échéant, l'organe de maintien dans une position de chevauchement de la commissure pouce-index puis d'enrouler l'unique sangle autour de la main. De plus, l'orthèse est adaptée à la morphologie de l'utilisateur grâce à l'organe de maintien souple. L'orthèse est également modulable d'une part du fait de sa symétrie gauche-droite et d'autre part du fait de la mise en place optionnelle de l'organe de maintien, la sangle pouvant être utilisée seule.

L'orthèse permet l'immobilisation juste nécessaire des segments concernés, c'est-à-dire l'immobilisation de la colonne ostéo-articulaire du pouce mais la liberté de l'interphalangienne du pouce. L'orthèse agit à la fois comme un produit antalgique et comme un moyen de prévention d'une aggravation, comme par exemple d'une luxation.

Il va de soi que l'invention n'est pas limitée aux modes de réalisation décrits ci-dessus à titre d'exemples mais qu'elle en embrasse au contraire toutes les variantes de réalisation couvertes par les revendications.

## Revendications

1. Orthèse destinée à être placée sur la main d'un utilisateur, notamment pour le traitement de la rhizarthrose, l'orthèse comprenant :
- un organe de maintien (2) agencé pour pouvoir être placé au niveau de la commissure pouce-index ;
- une sangle (3) placée autour de la main et autour de l'organe de maintien (2), pour assurer la retenue de cet organe de maintien (2) ;
**caractérisée en ce que** l'organe de maintien (2) présente la forme générale d'une selle de cheval, possédant une aile interne (5) et une aile externe (4) reliées par une bande de jonction (6) incurvée en U, l'organe de maintien étant agencé pour pouvoir être placé au niveau de la commissure pouce-index avec l'aile interne (5) contre la paume (15) et l'aile externe (4) contre la face dorsale (14) de la main, la bande de jonction (6) prenant appui d'une part sur la phalange proximale du pouce et d'autre part sur la phalange proximale de l'index afin de permettre le maintien d'un écartement entre le pouce et l'index, l'orthèse (1) étant agencée de sorte que l'os métacarpien du pouce soit emprisonné dans l'orthèse (1) entre la sangle (3) et l'organe de maintien (2).

2. Orthèse selon la revendication 1, **caractérisée en ce que** l'organe de maintien (2) et la sangle (3) sont deux pièces distinctes.

3. Orthèse selon la revendication 1 ou 2, **caractérisée en ce que** l'aile interne (5) de l'organe de maintien (2) est courbée vers l'extérieur en direction de son extrémité libre.

4. Orthèse selon l'une des revendications 1 à 3, **caractérisée en ce que** l'organe de maintien (2) est réalisé en une matière souple, telle que le silicone ou le SEBS (styrène éthylbutylène styrène).

5. Orthèse selon l'une des revendications 1 à 4, **caractérisée en ce que** l'organe de maintien (2) comporte une pièce en U rigide (23) située sensiblement à la jonction entre les ailes interne et externe (5, 4), ladite pièce en U (23) pouvant être déformée de façon à modifier l'écartement entre le pouce et l'index selon la morphologie de l'utilisateur.

6. Orthèse selon l'une des revendications 1 à 5, **caractérisée en ce qu'**elle comprend en outre une tige rigide (22) associée à l'organe de maintien (2) et/ou à la sangle (3) de sorte que, en position d'utilisation, ladite tige (22) s'étende sur la face dorsale (14) de la main sensiblement dans le prolongement de l'aile externe (4) de l'organe de maintien (2) et crée un appui contre la face dorsale de la main.

7. Orthèse selon la revendication 6, **caractérisée en ce que** la tige rigide (22) est réalisée en un matériau déformable de sorte qu'une personne puisse la plier ou l'incurver de façon à placer le poignet en légère flexion, en position d'utilisation de l'orthèse (1).

8. Orthèse selon la revendication 6 ou 7, **caractérisée en ce que** l'organe de maintien (2) comprend un logement (10) ménagé dans son aile externe (4) et destiné à recevoir une partie extrême de la tige rigide (22).

9. Orthèse selon l'une des revendications 1 à 8, **caractérisée en ce que** la sangle (3) présente, à plat, sensiblement la forme d'un T qui possède une première et une deuxième branches (16, 17) sensiblement dans le prolongement l'une de l'autre destinées à envelopper la main en étant rabattues contre la face dorsale (14) de la main, et une troisième branche (18) destinée à être rabattue vers la face dorsale (14) de la main en passant par la commissure pouce-index et en recouvrant l'organe de maintien (2).

10. Orthèse selon l'une des revendications 1 à 9, **caractérisée en ce que** la sangle (3) présente une dimension suffisante pour entourer également le poignet, en position d'utilisation.

11. Orthèse selon l'une des revendications 1 à 10, **caractérisée en ce que** la sangle (3) comporte au moins un élément de rigidification (20) qui, en position d'utilisation, s'étend sur la face dorsale (14) de la main sensiblement dans le prolongement de l'aile externe (4) de l'organe de maintien (2).

12. Orthèse selon l'une des revendications 1 à 11, **caractérisée en ce qu'**elle est agencée pour que, lorsqu'elle est portée par un utilisateur, la main de l'utilisateur équipée de l'orthèse (1) ait une inclinaison cubitale.

13. Orthèse selon l'une des revendications 1 à 12, **caractérisée en ce qu'**elle comprend en outre une barrette (24) rigide associée à la sangle (3), ladite barrette (24) s'étendant, en position d'utilisation, du côté intérieur de la main et de l'avant-bras sensiblement selon l'axe de l'avant-bras, présentant une longueur suffisante pour s'étendre de part et d'autre de l'articulation du poignet, et possédant une forme courbée épousant le relief du poignet et de la paume de la main.

## Patentansprüche

1. Orthese, die dazu ausgelegt ist, um auf der Hand eines Benutzers positioniert zu werden, insbesondere zur Behandlung der Rhizarthrose, wobei die Orthese folgendes umfasst:
- ein Rückhalteorgan (2), das angeordnet ist, um auf der Ebene der Verbindungsstelle zwischen Daumen und Zeigefinger positioniert zu werden;
- einen Gurt (3), der um die Hand und um das Rückhalteelements (2) positioniert ist, um den Rückhalt dieses Rückhalteorgan (2) sicherzustellen;
- **dadurch gekennzeichnet, dass** das Rückhalteorgan (2) die allgemeine Form eines Pferdesattels aufweist, umfassend einen inneren Flügel (5) und einen äußeren Flügel (4), die durch ein in Form eines "U" gebogenes Verbindungsband (6) verbunden sind, wobei das Rückhalteelement angeordnet ist, um auf der Ebene der Verbindungsstelle zwischen Daumen und Zeigefinger mit dem inneren Flügel (5) gegen die Handfläche (15) und dem äußeren Flügel (4) gegen die Rückseite (14) der Hand positioniert werden zu können, wobei das Verbindungsband (6) einerseits auf dem proximalen Fingerglied des Daumens und andererseits auf dem proximalen Fingerglied des Zeigefingers aufliegt, um die Beibehaltung eines Abstands zwischen dem Daumen und dem Zeigefinger zu ermöglichen, wobei die Orthese (1) derart angeordnet ist, dass der Mittelhandknochen des Daumens in der Orthese (1) zwischen dem Gurt (3) und dem Rückhalteorgan (2) eingeschlossen ist.

2. Orthese nach Anspruch 1, **dadurch gekennzeichnet, dass** das Rückhalteorgan (2) und der Gurt (3) zwei verschiedene Teile sind.

3. Orthese nach Anspruch 1 oder 2, **dadurch gekennzeichnet, dass** der innere Flügel (5) des Rückhalteorgans (2) nach außen in Richtung seines freien Endes gekrümmt ist.

4. Orthese nach einem der Ansprüche 1 bis 3, **dadurch gekennzeichnet, dass** das Rückhalteorgan (2) aus einem weichen Material hergestellt ist, wie z.B. Silikon oder SEBS (Styrenethylbutylenstyren).

5. Orthese nach einem der Ansprüche 1 bis 4, **dadurch gekennzeichnet, dass** das Rückhalteorgan (2) ein Stück in Form eines starren U (23) umfasst, das sich im Wesentlichen an der Verbindung zwischen dem inneren und dem äußeren Flügel (5, 4) befindet, wobei das Stück in Form eines U (23) verformt werden kann, um den Abstand zwischen dem Daumen und dem Zeigefinder gemäß der Morphologie des Benutzers zu modifizieren.

6. Orthese nach einem der Ansprüche 1 bis 5, **dadurch gekennzeichnet, dass** sie außerdem einen starren Stift (22) umfasst, der mit dem Rückhalteorgan (2) und/oder dem Gurt (3) derart assoziiert ist, dass sich in der Verwendungsposition der Stift (22) auf der Rückseite (14) der Hand erstreckt, im Wesentlichen in der Verlängerung des äußeren Flügels (4) des Rückhalteorgans (2), und eine Auflage gegen die Rückseite der Hand bildet.

7. Orthese nach Anspruch 6, **dadurch gekennzeichnet, dass** der starre Schaft (22) aus einem verformbaren Material hergestellt ist, so dass eine Person ihn biegen oder krümmen kann, um das Handgelenk in der Verwendungsposition der Orthese (1) in einer leichte Biegung zu versetzten.

8. Orthese nach Anspruch 6 oder 7, **dadurch gekennzeichnet, dass** das Rückhalteorgan (2) eine Lagerung (10) umfasst, die in seinem äußeren Flügel (4) angeordnet und ausgelegt ist, um einen Endteil des starren Schafts (22) aufzunehmen.

9. Orthese nach einem der Ansprüche 1 bis 8, **dadurch gekennzeichnet, dass** der Gurt (3) flach im Wesentlichen die Form eines T aufweist, das einen ersten und einen zweiten Zweig (16, 17) umfasst, im Wesentlichen in der Verlängerung voneinander, die ausgelegt sind, um die Hand zu umwickeln, wenn sie gegen die Rückseite (14) der Hand heruntergedrückt sind, und einen dritten Zweig (18), der ausgelegt ist, um gegen die Rückseite (14) der Hand heruntergedrückt zu werden, in dem er durch die Verbindungsstelle zwischen Daumen und Zeigefinger verläuft und das Rückhalteorgan (2) bedeckt.

10. Orthese nach einem der Ansprüche 1 bis 9, **dadurch gekennzeichnet, dass** der Gurt (3) eine ausreichende Abmessung aufweist, um in der Verwendungsposition auch das Handgelenk zu umgeben.

11. Orthese nach einem der Ansprüche 1 bis 10, **dadurch gekennzeichnet, dass** der Gurt (3) mindestens ein Versteifungselement (20) umfasst, das sich in der Verwendungsposition auf der Rückseite (14) der Hand erstreckt, im Wesentlichen in der Verlängerung des äußeren Flügels (4) des Rückhalteorgans (2).

12. Orthese nach einem der Ansprüche 1 bis 11, **dadurch gekennzeichnet, dass** sie so angeordnet ist, dass, wenn sie von einem Benutzer betragen wird, die Hand des Benutzers, der mit der Orthese (1) ausgestattet ist, eine Neigung des Ellenbogens aufweist.

13. Orthese nach einem der Ansprüche 1 bis 12, **dadurch gekennzeichnet, dass** sie außerdem einen starren Stab (24) umfasst, der mit dem Gurt (3) assoziiert ist, wobei sich der Stab (24) in der Verwendungsposition von der inneren Seite der Hand und des Unterarms im Wesentlichen gemäß der Achse des Unterarms erstreckt und eine ausreichende Länge aufweiset, um sich auf beiden Seiten des Handgelenks zu erstrecken, und eine gekrümmte Form aufweist, die sich dem Relief des Handgelenks und der Handfläche anpasst.

## Claims

1. An orthosis intended to be placed on the hand of a user, in particular to treat rhizarthrosis, the orthosis comprising:
- a supporting member (2) arranged to be able to be placed at the thumb-index commissure;
- a strap (3) to be placed around the hand and the supporting member (2), so as to retain said supporting member (2);
**characterized in that** the supporting member (2) has the general shape of a horse saddle, having an inner flange (5) and an outer flange (4) connected by a curved U-shaped junction band (6), the supporting member being arranged to be able to be placed at the thumb-index commissure with the inner flange (5) against the palm (15) and the outer flange (4) against the back surface (14) of the hand, the junction band (6) bearing on the one hand on the proximal phalange of the thumb and on the other hand on the proximal phalange of the index finger in order to enable a spacing to be maintained between the thumb and the index finger, the orthosis (1) being arranged such that the metacarpal bone of the thumb is trapped inside the orthosis (1) between the strap (3) and the supporting member (2).

2. The orthosis according to claim 1, **characterized in that** the supporting member (2) and the strap (3) are two separate pieces.

3. The orthosis according to claimant 1 or 2, **characterized in that** the inner flange (5) of the supporting member (2) is outwardly curved toward the free end thereof.

4. The orthosis according to one of claims 1 to 3, **characterized in that** the supporting member (2) is made from a flexible material, such as silicone or SEBS (styrene ethylene butylene styrene).

5. The orthosis according to one of claims 1 to 4, **characterized in that** the supporting member (2) comprises a rigid U-shaped piece (23) situated substantially at the junction between the inner and outer flanges (5, 4), said U-shaped piece (23) being able to be deformed so as to modify the spacing between the thumb and the index finger depending on the user's morphology.

6. The orthosis according to one of claims 1 to 5, **characterized in that** it also includes a rigid rod (22) associated with the supporting member (2) and/or the strap (3) such that, in the usage position, said rod (22) extends over the back surface (14) of the hand substantially in the extension of the outer flange (4) of the supporting member (2) and creates bearing against the back surface of the hand.

7. The orthosis according to claim 6, **characterized in that** the rigid rod (22) is made from a deformable material such that a person can bend or curve it so as to place the wrist slightly fluxed, in the usage position of the orthosis (1).

8. The orthosis according to claim 6 or 7, **characterized in that** the supporting member (2) comprises a housing (10) formed in its outer flange (4) and intended to receive an end portion of the rigid rod (22).

9. The orthosis according to one of claims 1 to 8, **characterized in that** the strap (3), when flat, is substantially in the shape of a T that has first and second branches (16, 17) substantially one in the extension of the other intended to surround the hand while being folded down against the back surface (14) of the hand, and the third branch (18) intended to be folded toward the back surface (14) of the hand while passing through the thumb-index commissure and covering the supporting member (2).

10. The orthosis according to one of claims 1 to 9, **characterized in that** the strap (3) has a sufficient size to also surround the wrist, in the usage position.

11. The orthosis according to one of claims 1 to 10, **characterized in that** the strap (3) comprises at least one stiffening element (20) which, in the usage position, extends over the back surface (14) of the hand substantially in the extension of the outer flange (4) of the supporting member (2).

12. The orthosis according to one of claims 1 to 11, **characterized in that** it is arranged so that, when it is worn by a user, the hand of the user equipped with the orthosis (1) has a cubital incline.

13. The orthosis according to one of claims 1 to 12, **characterized in that** it also comprises a rigid strip (24) associated with the strap (3), said strip (24) extending, in the usage position, from the inner side of the hand and forearm substantially along the axis of the forearm, having a sufficient length to extend on either side of the wrist joint, and having a curved shape fitting the relief of the wrist and the palm of the hand.
